# EUROPEAN PATENT SPECIFICATION

(11) **EP 4 512 435 B1**
(45) Date of publication and mention of the grant of the patent: **29.10.2025**
(21) Application number: 23192445.7
(22) Date of filing: 21.08.2023
(51) Int. Cl.: A61L 24/00, A61L 24/02, A61L 24/10, A61L 27/24, A61L 27/46, A61L 27/54, A61L 27/58, C08L 89/06

(54) **COMPOSITIONS OF DERIVATIVES OF O-PHOSPHO-L-SERINE, THEIR USE AND A PROCESS FOR PRODUCING THEREOF**
ZUSAMMENSETZUNGEN VON O-PHOSPHO-L-SERIN-DERIVATEN, IHRE VERWENDUNG UND VERFAHREN ZU IHRER HERSTELLUNG
COMPOSITIONS DE DÉRIVÉS DE LA O-PHOSPHO-L-SÉRINE, LEUR UTILISATION ET LEUR PROCÉDÉ DE PRODUCTION

(43) Date of publication of application: 26.02.2025
(73) Proprietor: Sirona Dental Systems GmbH, 64625 Bensheim (DE); DENTSPLY SIRONA Inc., York, PA 17401 (US)
(72) Inventor: CARTIER, Régis, Dr., 64625 Bensheim (DE)
(74) Representative: Crow, Martin

(56) References cited:
- WO-A1-2010/056811
- WO-A2-2012/158527
- US-A1- 2012 082 705
- ALINA KIRILLOVA ET AL: "Bioinspired Mineral-Organic Bioresorbable Bone Adhesive", ADVANCED HEALTHCARE MATERIALS, WILEY - V C H VERLAG GMBH & CO. KGAA, DE, vol. 7, no. 17, 25 June 2018 (2018-06-25), pages n/a, XP072463163, ISSN: 2192-2640, DOI: 10.1002/ADHM.201800467
- NORTON MICHAEL R ET AL: "Bone glue - The final frontier for fracture repair and implantable device stabilization", INTERNATIONAL JOURNAL OF ADHESION AND ADHESIVES, ELSEVIER, AMSTERDAM, NL, vol. 102, 15 May 2020 (2020-05-15), XP086245211, ISSN: 0143-7496, [retrieved on 20200515], DOI: 10.1016/J.IJADHADH.2020.102647

## Description

### BACKGROUND OF THE INVENTION

Use of O-Phospho-L-serine in dental implant stabilization has been known for several years.

The prior art compositions comprising O-Phospho-L-serine have low biocompatibility and very often demonstrate low or moderate bone connection. They have been described, besides others, in the following publications:
(1) Pujari-Palmer, M.; Guo, H.; Wenner, D.; Autefage, H.; Spicer, C. D.; Stevens, M. M.; Omar, O.; Thomsen, P.; Edén, M.; Insley, G.; Procter, P.; Engqvist, H. A Novel Class of Injectable Bioceramics That Glue Tissues and Biomaterials. Materials 2018, 11 (12), 2492. https://doi.org/10.3390/ma11122492.
(2) Liu, X.; Pujari-Palmer, M.; Wenner, D.; Procter, P.; Insley, G.; Engqvist, H. Adhesive Cements That Bond Soft Tissue Ex Vivo. Materials 2019, 12 (15), 2473. https://doi.org/10.3390/ma12152473.
(3) Kirillova, A.; Kelly, C.; Windheim, N.; Gall, K. Bioinspired Mineral-Organic Bioresorbable Bone Adhesive. Adv. Healthc. Mater. 2018, 7 (17), 1800467. https://doi.org/10.1002/adhm.201800467.
(4) Farrar, D. F. Bone Adhesives for Trauma Surgery: A Review of Challenges and Developments. Int. J. Adhes. Adhes. 2012, 33, 89-97. https://doi.org/10.1016/j.ijadhadh.2011.11.009.
(5) Norton, M. R.; Kay, G. W.; Brown, M. C.; Cochran, D. L. Bone Glue - The Final Frontier for Fracture Repair and Implantable Device Stabilization. Int. J. Adhes. Adhes. 2020, 102, 102647. https://doi.org/10.1016/j.ijadhadh.2020.102647.
(6) Böker, K. O.; Richter, K.; Jäckle, K.; Taheri, S.; Grunwald, I.; Borcherding, K.; von Byern, J.; Hartwig, A.; Wildemann, B.; Schilling, A. F.; Lehmann, W. Current State of Bone Adhesives-Necessities and Hurdles. Materials 2019, 12 (23), 3975. https://doi.org/10.3390/ma12233975.
(7) Cochran, D. L.; Jones, A.; Sugita, R.; Brown, M. C.; Guda, T.; Prasad, H.; Ong, J. L.; Pollack, A.; Kay, G. W. Immediate Dental Implant Stabilization in a Canine Model Using a Novel Mineral-Organic Adhesive: 4-Month Results. Int. J. Oral Maxillofac. Implants 2020, 35 (1), 39-51. https://doi.org/10.11607/jomi.7891.
(8) Reinstorf, A.; Hempel, U.; Olgemöller, F.; Domaschke, H.; Schneiders, W.; Mai, R.; Stadlinger, B.; Rösen-Wolff, A.; Rammelt, S.; Gelinsky, M.; Pompe, W. O-Phospho-L-Serine Modified Calcium Phosphate Cements - Material Properties, in Vitro and in Vivo Investigations. Mater. Werkst. 2006, 37 (6), 491-503. https://doi.org/10.1002/mawe.200600026.

US 2012/083705 A1 discloses a self-setting bone cement comprising a mixture of TTCP (tetracalcium phosphate) (particle size between 10-100 microns), collagen and OPLS (O-phospho-L-serine). The cement presents good bone integration and suitable adhesive properties for the application as bone cement.

In the field of dental applications, bone growth products and adhesive materials each have their own unique advantages and disadvantages. Bone growth products are typically well-tolerated by the body, making them highly biocompatible. They provide a scaffold for new bone growth, a crucial factor for successful bone regeneration. Additionally, these materials maintain their volume over time, which is important for the success of the procedure. They have been widely used and studied, with many clinical trials demonstrating their effectiveness.

However, there are also some drawbacks to consider. Some of these products are derived from animals, which may raise concerns about disease transmission. Although the risk is extremely low due to rigorous processing and sterilization, it is still a factor to consider. These products can also be more expensive than some other grafting materials. Additionally, they may resorb slowly, which can be a disadvantage in some cases where faster bone regeneration is desired.

On the other hand, adhesive materials used in dental applications offer strong adhesion, providing a robust bond that can withstand the forces in the oral environment. Like bone growth products, they are well-tolerated by the body, making them biocompatible. They are versatile and can be used with a variety of materials, including metal, ceramic, and natural bone. Another advantage is their rapid setting, which can be beneficial in surgical settings.

However, adhesive materials used in dental applications, while beneficial, do come with their own set of potential drawbacks. While many of these materials are designed to be biocompatible, pH issues occur, leading to inflammation reactions and cytotoxic effects.

Another issue is the degradation of the adhesive over time. If the product degrades too quickly, it will lead to a loss of bond strength and the need for replacement or repair. It is why the degradation of adhesive material is designed to be slow, resulting in the replacement of the natural bone taking a lot of time, which can, however, increase the risk of complications and overloaded implant.

The application of these adhesives can also represent a challenge to apply them precisely, especially in the limited space of the oral cavity. This could potentially lead to issues with the fit and positioning of the dental implant.

It is an object of the present invention to overcome the above shortcomings.

### Terms and Abbreviations used in the present invention:

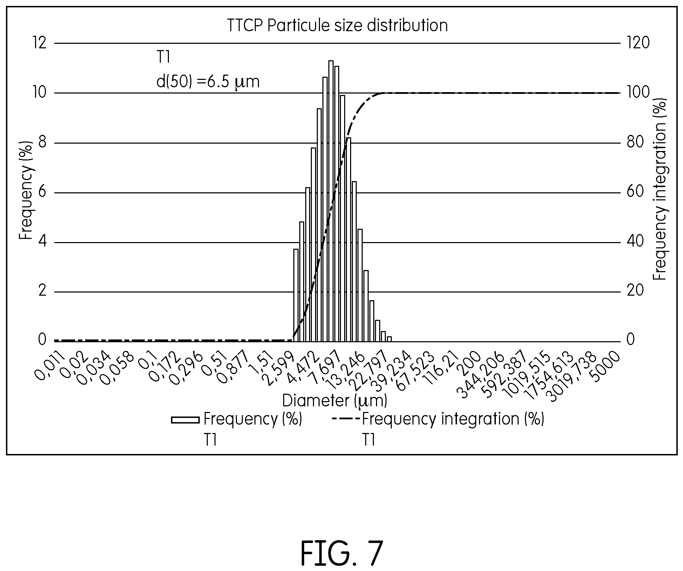

**Gly** = Glycine (amino acid) of the Formula (0) : 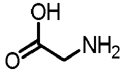
**OPLS** = O-phospho-L-serine of the Formula (II) : 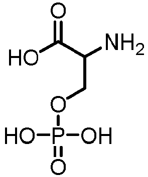
**OPLS-Gly** = O-phospho-L-serine modified with Glycine (OPLS-Gly; O-phospho-L-serine-Glycine); a compound of the Formula (la): 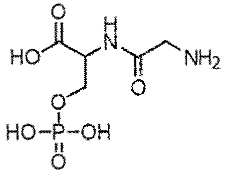
**ISQ=** Implant Stability Quotient; its value is in a scale from 1 to 100; it measures implant stability. The ISQ scale has a non-linear correlation with micromobility
**TTCP** = tetracalcium phosphate
**Xcoll** = crosslinked collagen
**Ca/P** = ratio of Calcium/Phosphorus atoms in tetracalcium phosphate
**L/P** = Liquid/Powder ratio, e.g. Water/Powder ratio
**BGM** = bone graft material
**Adhesive/mineral ratio** = ratio between the compound of the Formula (I) or a compound of the Formula (la) and tetracalcium phosphate
**Bio-Oss^{®}=** Spongious bone substitute from bovine derivatives of a company called Geistlich
**Setting time** = defined with a Gillmore needle, the setting time represents the time elapsed from mixing to the time at which no indentation can be detected on the mixture surface. The initial setting time and final setting time were determined corresponding to the ASTM C266-03 (2008) and ISO9917.
**d(50)** = A median diameter of a particle of a powder. 50% of the particles are below the size d and 50 % of the particles are above the size d (See Fig. 7).
**Drug** in the sense of the present invention is a medical drug.

### Description of the Figures

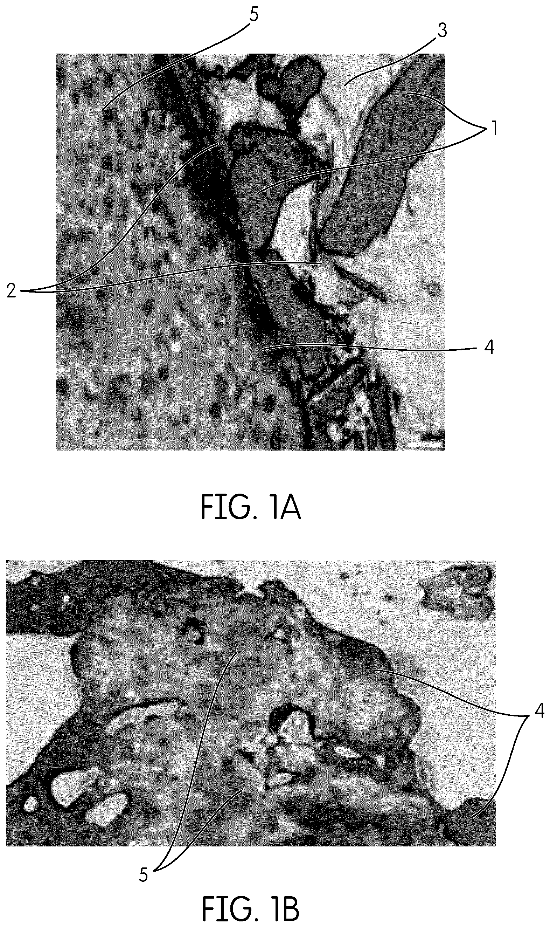

**Fig. 1A** depicts an example of histological result of a composition of a company called Paragon, a prior art composition. There are cytoplasms (1), nuclei (2) and extracellular matrix (3) .
   There is depicted the contact interface between the bone (4) and the bone graft material (5). The distribution of the staining represents the limited bone connection with the bone graft material. The bone graft material (5) remains separated from the bone (4).
**Fig. 1B** depicts histological results: an excellent bone connection with the composition of the present invention (a bone graft material) (5), after 12 weeks of bone-to-implant contact.
   The histological staining demonstrates the difference in comparison with the Fig.1a. The staining is mixed around the contact between the bone (4) and the bone graft material (5). Thus, the bone graft material (5) is well integrated into the structure of the bone (4).

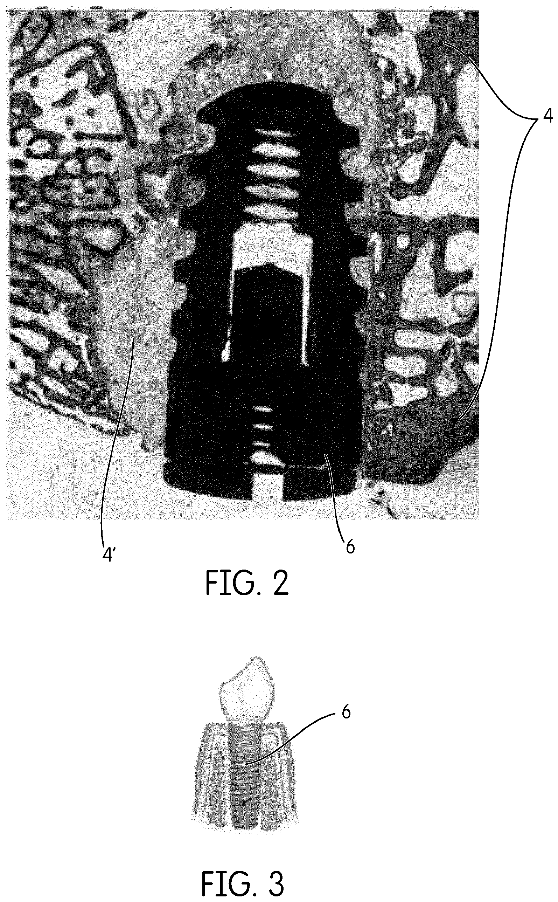

**Fig. 2** depicts histological results: the excellent bone connection with the composition of the present invention (a bone graft material) after 5 weeks of bone-to-implant contact. The bone graft material was placed with an implant (6) in an animal model for 5 weeks. The implant is illustrated in black, there is a bone (4); a new bone (4') is showing a good structural integration. The composition of the present invention, a bone graft material is integrated in the new bone (4').
**Fig. 3** depicts an implant (6) and the drill hole in case of a single root extraction.

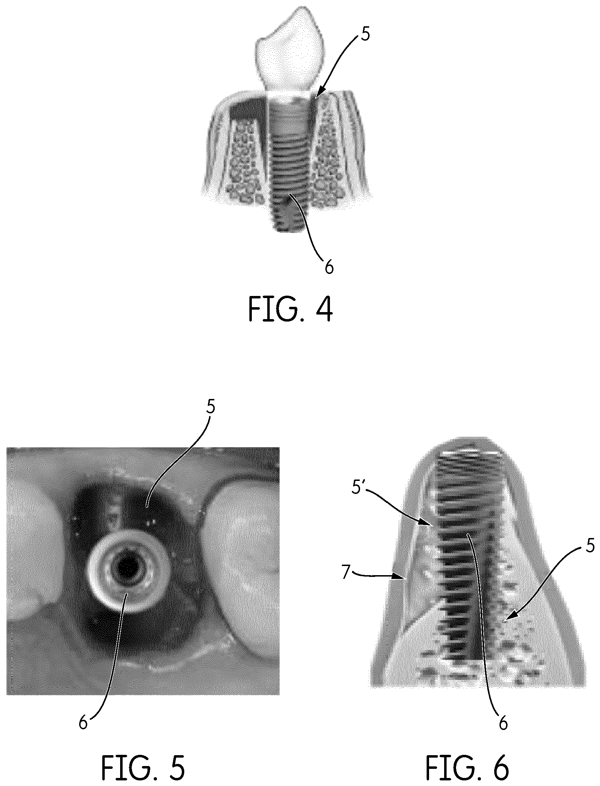

**Fig. 4****:** In case of a single root extraction, a composition of the present invention, a bone graft material (5) is needed to fill the gap between the placed implant (6) and the extraction socket; the healing period is estimated for 3-4 months. The placement of the restoration is possible after 4-6 months. The placement of a provisional restoration is possible within 48 hours.
**Fig. 5****:** For molar (multiple root teeth) extraction, the healing is estimated for 4-6 months with the use of a bone graft material to ensure and achieve stabilisation. The placement of restoration is possible after 6 months. The advantage of the composition of the present invention (a bone graft material) (5) is that it enables implant (6) stabilization in an ideal position.
**Fig. 6** depicts the use of the composition of the present invention (a bone graft material) (5) in combination with another bone graft material (BGM) (5'). In this application, the bone wall is resorbed, and a partial implant (6) fixation is needed. The healing is estimated for 4-6 months with bone graft material to rebuild the missing bone wall. Another bone graft material (5') is used with a membrane (7) to cover the bone graft initially. The placement of the restoration is possible after 4-6 months. The composition of the present invention (a bone graft material) (5) in combination with another bone graft material (5') ensures implant (6) stabilization in the ideal position and adds volume.
**Fig. 7** depicts tetracalcium phosphate particle size distribution. For the tetracalcium phosphate, the d(50) is preferably ≤ 10µm, which gives the composition good mechanical properties and appropriate setting time. d(50) is a median diameter of a particle of a powder. 50% of the particles are below the size d and 50 % of the particles are above the size d.

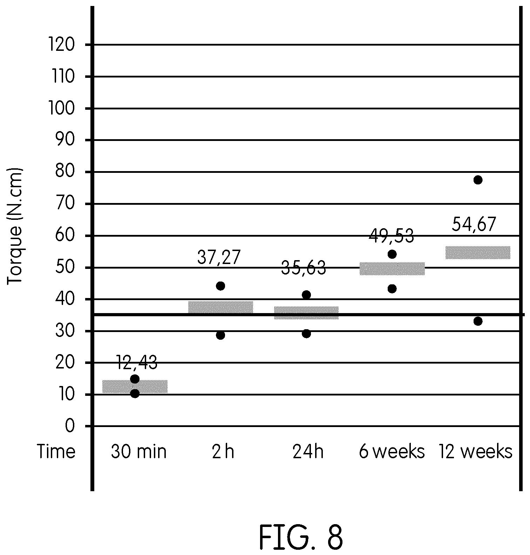

**Fig. 8** depicts results of the Reverse Torque (N.cm) test of the implant stabilized with the composition of the present invention i.e. the necessary torque to remove the implant. The Reverse Torque was measured after 30 min, 2 h, 24 h, 6 weeks and 12 weeks, calculated from the application of the composition of the present invention. Reverse Torque of 35 N.cm is a target value for immediate provisional loading. The dots represent the extremities of the standard deviation, while the bars represent the mean of the measurements.

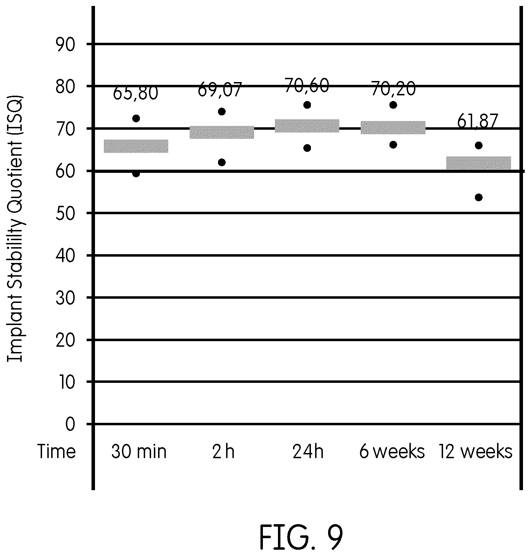

**Fig. 9** depicts the results of the ISQ test of the implant stabilized with the composition of the present invention after 30 min, 2 h, 24 h, 6 weeks and 12 weeks, calculated from the application of the composition of the present invention. ISQ of 60 is a target value for immediate provisional loading. The dots represent the extremities of the standard deviation, while the bars represent the mean of the measurements.
   In the above description of the Figures 1-9, the composition of the present invention is in particular the composition as defined in claim 2 and more preferably as defined in the Example 1.

### DETAILED DESCRIPTION OF THE INVENTION

It has been found that compositions based on O-phospho-L-serine modified with an alpha amino acid give surprisingly good results in respect of biocompatibility and bone connection.

It is an object of the present invention to provide a compound of Formula (Ia), 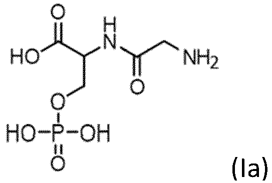 for use in the dental and medical practice.

The compound of the Formula I(a) is OPLS-Gly, i.e. O-Phospho-L-serine-Glycine.

It is an object of the present invention to provide a composition according to claim 1 comprising the compound of Formula (la), TTCP, a cross-linked collagen and water.

In the preferred embodiment of the present invention, the cross-linked collagen is a sugar cross-linked collagen.

In the preferred embodiment of the present invention, the cross-linked collagen is grinded and sieved to the particle size of 80 µm. The said particle size has a positive effect on the properties of the composition of the present invention, including absorption and biocompatibility.

In the preferred embodiment of the present invention, the composition comprises 40-70 weight % of TTCP, 15-40 weight % of the compound of the Formula (la), 10-30 weight % of water and 0.1-5.0 weight % of cross-linked collagen, based on the total weight of the composition.

In a more preferred embodiment of the present invention, the composition comprises 50-60 weight % of TTCP, 18-35 weight % of the compound of the Formula (la), 12-25 weight % of water and 1-2.5 weight % of cross-linked collagen, based on the total weight of the composition.

In a yet more preferred embodiment of the present invention, the composition comprises 58 weight % of TTCP, 23.7 weight % of the compound of the Formula (la), 17 weight % of water and 1 weight % of cross-linked collagen, based on the total weight of the composition.

In the preferred embodiment of the present invention, the weight ratio the compound of the Formula (la) and TTCP is 0.41, i.e. the adhesive/mineral ratio is 0.41, which gives good mechanical and adhesive properties to the composition. The above weight ratio also makes it possible to obtain a final setting time of less than or equal to 30 minutes.

In the preferred embodiment of the present invention, the liquid/powder ratio, i.e. the water/powder ratio is 0.21. Reduction in the liquid/powder ratio results in the reduction in the setting time.

The Ca/P ratio of the mineral part of the composition, i.e. tetracalcium phosphate, is 2, which is close to that of the natural bone. Tetracalcium phosphate is a mineral part of the composition.

For the TTCP, the d(50) is ≤ 10 µm, which gives the composition good mechanical properties and appropriate setting time. The Fig. 7 depicts particle size distribution of TTCP. In one of the embodiments of the present invention, the d(50) is 6.5 µm.

The particle diameter was measured by standard methods of the state of the art.

The composition as defined above for use in producing a bone graft.

The composition as defined above for use in producing a bone graft material. The composition as defined above in combination with another bone graft material for use in producing a bone graft.

The composition of the present invention can be used as an additive to a material used for tissue regeneration. In particular, it can be used as an adhesive in order to improve the mechanical stability of the used material. It can be also used to provide mechanical stability to another bone graft material. Furthermore, it can be used when a rapid dissolution or resorption of the material is needed in addition to stimulation of the formation of a new bone: to provide primary stability. The composition can be used in provisional restoration or a bone graft. Compared with Bio-Oss^{®}, which represents the state of the art in terms of bone substitutes for regenerative dentistry, this invention makes it possible to obtain a composition that can be modellable and provides immediate primary stability during implant fixation. Indeed, Bio-Oss^{®} does not provide immediate primary stability and is composed of granules that can move and, when resorbed, generate bone outgrowths.

Possible dental applications of the composition of the present invention include stabilization of a dental implant during an immediate implant placement procedure when there is not sufficient bone to support the implant. This could be applied for a single root or a molar extraction. The composition could also be used in combination with other bone graft materials.

Another possible application of the composition of the present invention is dental ridge preservation in which the composition of the present invention entirely fills the ridge due to its fluidity and subsequently acts as a stable filler after solidification. Hence, the composition would not move and provide volume stability.

Another possible application of the composition of the present invention is the treatment of vertical and horizontal bone loss, said treatment is not part of the invention. In these cases, as the composition of the present invention self-hardens rapidly, it may or may not be covered by a bio-membrane.

Another possible application of the composition of the present invention is the treatment of larger bone defects in oral and maxillofacial surgery in which the composition of the present invention is used to cover and stabilize a material used as a main filler of the defect, said method of treatment is not part of the invention. In this case, the solidified composition of the present invention would hold the filler material in place by providing a wall- and/or upper-shell-like structure.

Another possible application of the composition of the present invention is the structural stabilization of a soft bio-membrane comprising a synthetic or a natural polymer, wherein the soft bio-membrane is presented in a flexible form. The membrane could be covered by the composition of the present invention to confer mechanical rigidity upon solidification.

Another possible application of the composition of the present invention is the connection of rigid and soft materials including a natural or a synthetic bone and metals such as titanium and its alloys in a surgical procedure. The composition can strongly hold together two pieces of bone fragments, two metal surfaces or also a piece of a bone and a metal surface.

Another possible application of the composition of the present invention is the stabilization of bone fractures: the composition of the present invention can enhance or replace a surgical or nonsurgical treatment to fix and repair bone fractures, said method of treatment is not part of the invention.

Another possible application of the composition of the present invention is a delivery of a drug selected from the group comprising a growth factor drug, an anti-inflammatory drug, an antibiotic, other antimicrobial substances and substances supporting the healing process or their combination. The composition of the present invention contains the above drugs and acts as a delivery system. As such, the fluidity of the composition allows complete filling of a cavity to be treated and *in situ* hardening leads to sustained release of the drug to maintain its effect over a prolongated period, said method of treatment is not part of the invention. The drugs can also be pre-encapsulated in the composition of the present invention or in other types of materials. This results in a structured delivery system, allowing a controlled release delivery of at least one drug.

The composition of the present invention is produced by the following steps:
a. Providing the following powder components: O-Phospho-L-serine-Glycine, TTCP and a sugar cross-linked collagen.
b. Providing water.
c. Bringing the powder components of step a. and water into contact and mixing them to provide a composition.
**d.** Extruding the composition of step c., wherein the Ca/P ratio of the mineral part of the composition is 2 and wherein the d(50) of the tetracalcium phosphate (TTCP) is ≤10µm.

### EXAMPLES

### Example 1: Composition preparation

First, the single powder components were weighed *i.e.* TTCP (293 mg, 0.80 mmol), OPLS-Gly (120 mg, 0.50 mmol) and a sugar cross-linked collagen (5 mg) and then were introduced in a capsule. Before weighing, the sugar cross-linked collagen was grinded and sieved to the particle size of 80 µm. The d(50) of TTCP was 6.5 µm. Then, into a separate compartment of the capsule, water (87 µl) was provided. The powder compoments and water were brought into contact by activating the capsule and mixed for 30 seconds to obtain the composition with the appearance of white putty. Finally, the composition was extruded using a capsule extruder.

## Claims

1. A composition comprising:
a. O-Phospho-L-serine-Glycine
b. Tetracalcium phosphate
c. A sugar cross-linked collagen
d. Water,
wherein the Ca/P ratio of the mineral part of the composition is 2 and wherein the d(50) of tetracalcium phosphate is ≤ 10µm.

2. The composition of claim 1, wherein the composition comprises 40-70 weight % of tetracalcium phosphate, 15-40 weight % of O-Phospho-L-serine-Glycine, 10-30 weight % of water and 0.1-5.0 weight % of sugar cross-linked collagen, based on the total weight of the composition.

3. The composition of claim 2, wherein the weight ratio of O-Phospho-L-serine-Glycine and tetracalcium phosphate is 0.41.

4. The composition of claims 1 -3, wherein the liquid /powder ratio of the composition is 0.21.

5. A process for producing the composition according to claims 1-4, comprising the following steps:
a. Providing the following powder components: O-Phospho-L-serine-Glycine, tetracalcium phosphate and a sugar cross-linked collagen
b. Providing water.
c. Bringing the powder components of step a. and water into contact and mixing them to provide a composition.
d. Extruding the composition of step c.,
wherein the Ca/P ratio of the mineral part of the composition is 2 and wherein the d(50) of tetracalcium phosphate ≤ 10µm.

6. The composition of claims 1-4 for use in producing a bone graft material.

7. The composition of claims 1-4 for use in producing a bone graft.

8. The composition of claims 1-4 in combination with another bone graft material for use in producing a bone graft.

9. The composition of claims 1-4 for use in the stabilization of a dental implant.

10. The composition of claims 1-4 for use in the dental ridge preservation.

11. The composition of claims 1-4 for use in the treatment of vertical and horizontal bone loss.

12. The composition of claims 1-4 for use in the treatment of larger bone defects.

13. The composition of claims 1-5 for use in the structural stabilization of a soft bio -membrane comprising a synthetic or a natural polymer.

14. The composition of claims 1-4 for use in the connection of rigid and soft materials including a natural or a synthetic bone and metals such as titanium and its alloys in a surgical procedure.

15. The composition of claims 1-4 for use in the stabilization of bone fractures.

16. The composition of claims 1-4 for use in the controlled release delivery of at least one drug.

17. O-Phospho-L-serine-Glycine for use in the dental and medical practice.

## Patentansprüche

1. Zusammensetzung, umfassend:
a. O-Phospho-L-Serin-Glycin
b. Tetracalciumphosphat
c. ein zuckervernetztes Kollagen
d. Wasser,
wobei das Ca/P-Verhältnis des mineralischen Teils der Zusammensetzung 2 beträgt und wobei das d(50) von Tetracalciumphosphat ≤ 10 µm ist.

2. Zusammensetzung nach Anspruch 1, wobei die Zusammensetzung 40-70 Gew.-% Tetracalciumphosphat, 15-40 Gew.-% O-Phospho-L-Serin-Glycin, 10-30 Gew.-% Wasser und 0,1-5,0 Gew.-% zuckervernetztes Kollagen, bezogen auf das Gesamtgewicht der Zusammensetzung, umfasst.

3. Zusammensetzung nach Anspruch 2, wobei das Gewichtsverhältnis von O-Phospho-L-Serin-Glycin und Tetracalciumphosphat 0,41 beträgt.

4. Zusammensetzung nach den Ansprüchen 1-3, wobei das Flüssigkeits-/Pulver-Verhältnis der Zusammensetzung 0,21 beträgt.

5. Verfahren zur Herstellung der Zusammensetzung nach den Ansprüchen 1-4, umfassend die folgenden Schritte:
a. Bereitstellen der folgenden Pulverkomponenten: O-Phospho-L-Serin-Glycin, Tetracalciumphosphat und ein zuckervernetztes Kollagen
b. Bereitstellen von Wasser,
c. Inkontaktbringen der Pulverkomponenten aus Schritt a. und Wasser und Mischen derselben, um eine Zusammensetzung bereitzustellen,
d. Extrudieren der Zusammensetzung aus Schritt c.,
wobei das Ca/P-Verhältnis des mineralischen Teils der Zusammensetzung 2 beträgt und wobei das d(50) von Tetracalciumphosphat ≤ 10 µm.

6. Zusammensetzung nach den Ansprüchen 1-4 zur Verwendung beim Herstellen eines Knochentransplantatmaterials.

7. Zusammensetzung nach den Ansprüchen 1-4 zur Verwendung beim Herstellen eines Knochentransplantats.

8. Zusammensetzung nach den Ansprüchen 1-4 in Kombination mit einem anderen Knochentransplantatmaterial zur Verwendung beim Herstellen eines Knochentransplantats.

9. Zusammensetzung nach den Ansprüchen 1-4 zur Verwendung bei der Stabilisierung eines Zahnimplantats.

10. Zusammensetzung nach den Ansprüchen 1-4 zur Verwendung bei der Kieferkammerhaltung.

11. Zusammensetzung nach den Ansprüchen 1-4 zur Verwendung bei der Behandlung von vertikalem und horizontalem Knochenverlust.

12. Zusammensetzung nach den Ansprüchen 1-4 zur Verwendung bei der Behandlung größerer Knochendefekte.

13. Zusammensetzung nach den Ansprüchen 1-5 zur Verwendung bei der strukturellen Stabilisierung einer weichen Biomembran, die ein synthetisches oder ein natürliches Polymer umfasst.

14. Zusammensetzung nach den Ansprüchen 1-4 zur Verwendung bei der Verbindung von starren und weichen Materialien, einschließlich eines natürlichen oder synthetischen Knochens und Metallen wie Titan und seinen Legierungen bei einem chirurgischen Eingriff.

15. Zusammensetzung nach den Ansprüchen 1-4 zur Verwendung bei der Stabilisierung von Knochenbrüchen.

16. Zusammensetzung nach den Ansprüchen 1-4 zur Verwendung bei der Abgabe mindestens eines Arzneimittels mit kontrollierter Freisetzung.

17. O-Phospho-L-Serin-Glycin zur Verwendung in der zahnärztlichen und medizinischen Praxis.

## Revendications

1. Composition, comprenant :
a. de l'O-phospho-L-sérine-glycine
b. du phosphate tétracalcique
c. du collagène réticulé par un sucre
d. de l'eau,
dans laquelle le rapport Ca/P de la partie minérale de la composition est de 2 et dans laquelle le d(50) du phosphate tétracalcique est ≤ 10 µm.

2. Composition de la revendication 1, ladite composition comprenant 40 à 70 % en poids de phosphate tétracalcique, 15 à 40 % en poids d'O-phospho-L-sérine-glycine, 10 à 30 % en poids d'eau et 0,1 à 5,0 % en poids de collagène réticulé par un sucre, sur la base du poids total de la composition.

3. Composition de la revendication 2, dans laquelle le rapport en poids d'O-phospho-L-sérine-glycine et de phosphate tétracalcique est de 0,41.

4. Composition des revendications 1 à 3, dans laquelle le rapport liquide/poudre de la composition est de 0,21.

5. Processus permettant la production de la composition selon les revendications 1 à 4, comprenant les étapes suivantes :
a. la fourniture des composants en poudre suivants : l'O-phospho-L-sérine-glycine, le phosphate tétracalcique et un collagène réticulé par un sucre,
b. la fourniture d'eau,
c. la mise en contact des composants en poudre de l'étape a. et de l'eau et le mélange de ceux-ci pour obtenir une composition,
d. l'extrusion de la composition de l'étape c.,
dans lequel le rapport Ca/P de la partie minérale de la composition est de 2 et dans lequel le d(50) de phosphate tétracalcique ≤ 10 µm.

6. Composition des revendications 1 à 4, destinée à être utilisée dans la production d'un matériau de greffon osseux.

7. Composition des revendications 1 à 4, destinée à être utilisée dans la production d'un greffon osseux.

8. Composition des revendications 1 à 4 en combinaison avec un autre matériau de greffon osseux, destinée à être utilisée dans la production d'un greffon osseux.

9. Composition des revendications 1 à 4, destinée à être utilisée dans la stabilisation d'un implant dentaire.

10. Composition des revendications 1 à 4, destinée à être utilisée dans la préservation de crêtes dentaires.

11. Composition des revendications 1 à 4, destinée à être utilisée dans le traitement de la perte osseuse verticale et horizontale.

12. Composition des revendications 1 à 4, destinée à être utilisée dans le traitement de défauts osseux plus importants.

13. Composition des revendications 1 à 5, destinée à être utilisée dans la stabilisation structurelle d'une biomembrane molle comprenant un polymère synthétique ou naturel.

14. Composition des revendications 1 à 4, destinée à être utilisée dans la liaison de matériaux rigides et mous comprenant un os naturel ou synthétique et des métaux tels que le titane et ses alliages dans une procédure chirurgicale.

15. Composition des revendications 1 à 4, destinée à être utilisée dans la stabilisation de fractures osseuses.

16. Composition des revendications 1 à 4, destinée à être utilisée dans l'administration à libération contrôlée d'au moins un médicament.

17. O-phospho-L-sérine-glycine destinée à être utilisée dans la pratique dentaire et médicale.
